# EUROPEAN PATENT SPECIFICATION

(11) **EP 4 333 645 B1**
(45) Date of publication and mention of the grant of the patent: **05.03.2025**
(21) Application number: 22747178.6
(22) Date of filing: 23.06.2022
(51) Int. Cl.: A23L 27/30, C12P 19/18

(54) **MOGROSIDE COMPOUNDS AND THEIR COMESTIBLE USE**
MOGROSIDVERBINDUNGEN UND DEREN VERWENDUNG ALS LEBENSMITTEL
COMPOSÉS MOGROSIDES ET LEURS UTILISATIONS ET LEUR UTILISATION COMESTIBLE

(30) Priority: 29.06.2021 US 202163216014 P
(43) Date of publication of application: 13.03.2024
(73) Proprietor: Firmenich Incorporated, Plainsboro, NJ 08536 (US)
(72) Inventor: NORIEGA, Chris Edano, San Diego, California 92121 (US); MANAM, Rama Rao, San Diego, California 92121 (US); WILLIAMS, Mark, San Diego, California 92121 (US); DITSCHUN, Tanya, San Diego, California 92121 (US)
(74) Representative: Strych, Sebastian
(86) International application number: PCT/US2022/034628
(87) International publication number: WO 2023/278226

(56) References cited:
- WO-A1-2020/097588
- US-A1- 2011 027 413
- US-A1- 2018 346 953
- US-A1- 2019 071 705
- US-A1- 2020 054 058
- MAXIM ITKIN ET AL: "The biosynthetic pathway of the nonsugar, high-intensity sweetener mogroside V from Siraitia grosvenorii", PROCEEDINGS OF THE NATIONAL ACADEMY OF SCIENCES, vol. 113, no. 47, 7 November 2016 (2016-11-07), pages E7619 - E7628, XP055578320, ISSN: 0027-8424, DOI: 10.1073/pnas.1604828113

## Description

### TECHNICAL FIELD

The present disclosure generally relates to certain mogroside compounds and the use of such compounds as sweeteners or to enhance sweetness when used in combination with other sweeteners. In certain aspects, the disclosure provides ingestible compositions and that include such mogroside compounds. In some embodiments, such ingestible compositions comprise monk fruit juice, monk fruit extract, or a combination thereof. In some related aspects, the ingestible compositions are, or are included within, various flavored products, such as food products, beverage products, or oral care products.

### DESCRIPTION OF RELATED ART

The taste system provides sensory information about the chemical composition of the external world. Taste transduction is one of the more sophisticated forms of chemically triggered sensation in animals. Signaling of taste is found throughout the animal kingdom, from simple metazoans to the most complex of vertebrates. Mammals are believed to have five basic taste modalities: sweet, bitter, sour, salty, and umami.

Sweetness is the taste most commonly perceived when eating foods rich in sugars. Mammals generally perceive sweetness to be a pleasurable sensation, except in excess. Caloric sweeteners, such as sucrose and fructose, are the prototypical examples of sweet substances. Although a variety of no-calorie and low-calorie substitutes exist, these caloric sweeteners are still the predominant means by which comestible products induce the perception of sweetness upon consumption.

Metabolic disorders and related conditions, such as obesity, diabetes, and cardiovascular disease, are major public health concerns throughout the world. And their prevalence is increasing at alarming rates in almost every developed country. Caloric sweeteners are a key contributor to this trend, as they are included in various packaged food and beverage products to make them more palatable to consumers. In many cases, no-calorie or low-calorie substitutes can be used in foods and beverages in place of sucrose or fructose. Even so, these compounds impart sweetness differently from caloric sweeteners, and many consumers fail to view them as suitable alternatives. Moreover, such compounds may be difficult to incorporate into certain products. In some instances, they may be used as partial replacements for caloric sweeteners, but their mere presence can cause many consumers to perceive unpleasant off-tastes including, astringency, bitterness, and metallic and licorice tastes. Thus, currently available lower-calorie sweeteners face certain challenges to their adoption. Thus, there is a continuing need to discover and develop no-calorie or low-calorie sweeteners that may be more acceptable to consumers.

US 2020/0054058 A1, US 2018/0346953 A1, WO 2020/097588 A1 and US 2019/0071705 A1 disclose sweet tasting triterpene glycosides produced by the enzymatic modification of mogrol and/ or mogrosides.

### SUMMARY

The present disclosure relates to the discovery that a particular mogroside compound, which is represented by the chemical structure 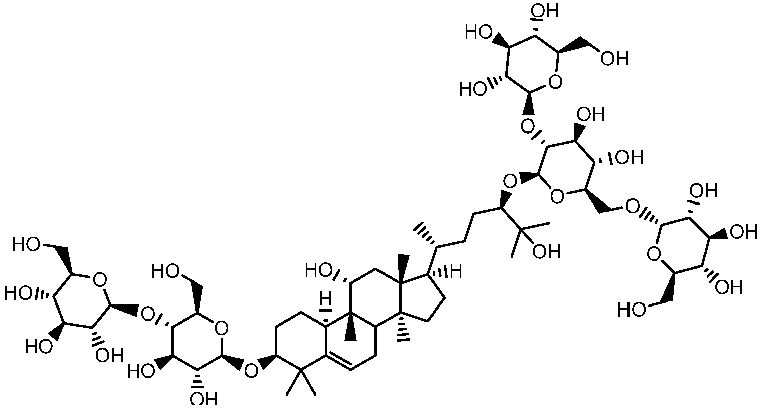 imparts certain beneficial taste properties, such as, for example, imparting a clean and natural sweetness to ingestible compositions when used alone or in combination with other sweeteners and flavor-modifying compounds. As used herein, the compound is referred to as the "MC1 compound."

In a first aspect, the disclosure provides sweetening compounds, which are the MC1 compound or comestibly acceptable salts thereof.

In a second aspect, the disclosure provides uses of the sweetening compounds of the first aspect to modify the flavor of or to impart a flavor to, such as to impart sweetness to, an ingestible composition. The disclosure provides related methods of modifying a flavor of or imparting a flavor to, such as to impart sweetness to, an ingestible composition, the method comprising introducing a sweetening compound of the first aspect to an ingestible composition. In some embodiments, the sweetening compounds are used in or introduced to the ingestible composition in a concentration ranging from 0.01 ppm to 1000 ppm. In some embodiments, the ingestible composition comprises a juice of an extract of monk fruit.

In a third aspect, the disclosure provides uses of the sweetening compounds of the first aspect to reduce the caloric content of an ingestible composition. The disclosure provides related methods of reducing the caloric content of an ingestible composition, the method comprising introducing a sweetening compound of the first aspect to an ingestible composition. In some embodiments, such calorie reduction occurs by permitting the reduced use of caloric sweeteners, such as sucrose, fructose, or glucose. In some embodiments, the sweetening compounds are used in or introduced to the ingestible composition in a concentration ranging from 0.01 ppm to 1000 ppm. In some embodiments, the ingestible composition comprises a juice of an extract of monk fruit.

In a fourth aspect, the disclosure provides ingestible compositions, which comprise sweetening compounds of the first aspect. In some embodiments, the ingestible composition comprises the sweetening compounds at a concentration ranging from 0.01 ppm to 1000 ppm. In some embodiments, the ingestible composition comprises a juice of an extract of monk fruit. In some embodiments, the ingestible composition comprises other sweeteners, flavor modifiers, mouthfeel enhancers, and the like.

In a fifth aspect, the disclosure provides flavored products, which comprise or consist of the ingestible compositions of the third aspect. In some embodiments, the flavored products are food products, beverage products, or oral care products.

Further aspects, and embodiments thereof, are set forth below in the Detailed Description, the Drawings, the Abstract, and the Claims.

### BRIEF DESCRIPTION OF THE DRAWINGS

The following drawings are provided for purposes of illustrating various embodiments of the compositions and methods disclosed herein. The drawings are provided for illustrative purposes only, and are not intended to describe any preferred compositions or preferred methods, or to serve as a source of any limitations on the scope of the claimed inventions.

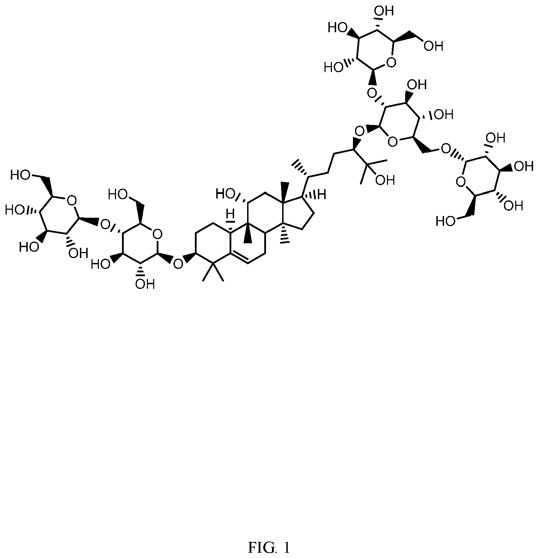

FIG. 1 shows a chemical formula of the MC1 compound.

### DETAILED DESCRIPTION

The following Detailed Description sets forth various aspects and embodiments provided herein. The description is to be read from the perspective of the person of ordinary skill in the relevant art. Therefore, information that is well known to such ordinarily skilled artisans is not necessarily included.

### Definitions

The following terms and phrases have the meanings indicated below, unless otherwise provided herein. This disclosure may employ other terms and phrases not expressly defined herein. Such other terms and phrases have the meanings that they would possess within the context of this disclosure to those of ordinary skill in the art. In some instances, a term or phrase may be defined in the singular or plural. In such instances, it is understood that any term in the singular may include its plural counterpart and vice versa, unless expressly indicated to the contrary.

As used herein, "solvate" means a compound formed by the interaction of one or more solvent molecules and one or more compounds described herein. In some embodiments, the solvates are comestibly acceptable solvates, such as hydrates.

As used herein, the "ppm" refers to a concentration of mg/kg, based on the total weight of the composition.

A "sweetener" refers to a compound or ingestibly acceptable salt thereof that elicits a detectable sweet taste in a subject, e.g., a compound that activates the T1R2 and T1R3 taste receptors in vivo or in vitro.

A "bitter tastant" refers to a compound or ingestibly acceptable salt thereof that elicits a detectable bitter taste in a subject, e.g., a compound that activates one or more T2R taste receptors in vivo or in vitro.

As used herein, the singular forms "a," "an," and "the" include plural referents unless the context clearly dictates otherwise. For example, reference to "a substituent" encompasses a single substituent as well as two or more substituents, and the like.

As used herein, "for example," "for instance," "such as," or "including" are meant to introduce examples that further clarify more general subject matter. Unless otherwise expressly indicated, such examples are provided only as an aid for understanding embodiments illustrated in the present disclosure, and are not meant to be limiting in any fashion. Nor do these phrases indicate any kind of preference for the disclosed embodiment.

As used herein, "comprise" or "comprises" or "comprising" or "comprised of" refer to groups that are open, meaning that the group can include additional members in addition to those expressly recited. For example, the phrase, "comprises A" means that A must be present, but that other members can be present too. The terms "include," "have," and "composed of" and their grammatical variants have the same meaning. In contrast, "consist of" or "consists of" or "consisting of" refer to groups that are closed. For example, the phrase "consists of A" means that A and only A is present.

As used herein, "optionally" means that the subsequently described event(s) may or may not occur. In some embodiments, the optional event does not occur. In some other embodiments, the optional event does occur one or more times.

As used herein, "or" is to be given its broadest reasonable interpretation, and is not to be limited to an either/or construction. Thus, the phrase "comprising A or B" means that A can be present and not B, or that B is present and not A, or that A and B are both present. Further, if A, for example, defines a class that can have multiple members, e.g., A₁ and A₂, then one or more members of the class can be present concurrently.

Chemical structures are often shown using the "skeletal" format, such that carbon atoms are not explicitly shown, and hydrogen atoms attached to carbon atoms are omitted entirely. For example, the structure 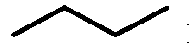 represents butane (i.e., n-butane). Furthermore, aromatic groups, such as benzene, are represented by showing one of the contributing resonance structures. For example, the structure 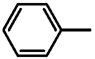 represents toluene.

Other terms are defined in other portions of this description, even though not included in this subsection.

### Mogroside Compound

In certain aspects, the disclosure provides sweetening compounds, which are a compound of the following formula or a comestibly acceptable salt thereof: 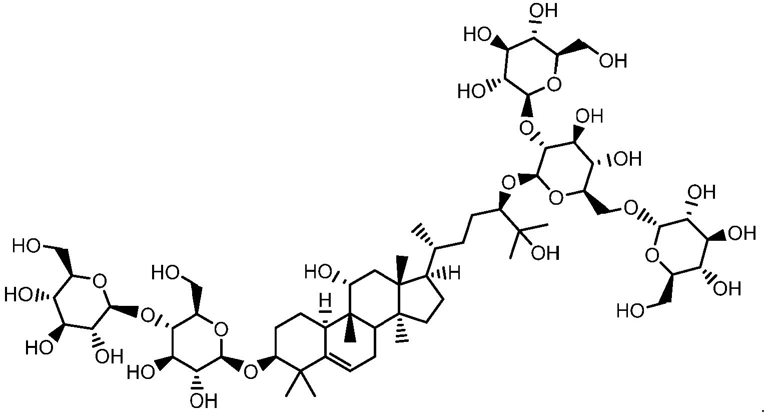 The compound of the above formula is also described herein as (2S,3R,4S,5S,6R)-2-(((2R,3S,4R,5R,6R)-6-(((3S,9R,10R,11R,13R,14S,17R)-17-((2R,5R)-5-(((2S,3R,4S,5S,6R)-4,5-dihy droxy-3-(((2S,3R,4S,5S,6R)-3,4,5-trihydroxy-6-(hydroxymethyl)tetrahydro-2H-pyran-2-yl)oxy)-6-((((2S,3R,4S,5S,6R)-3,4,5-trihydroxy-6-(hydroxymethyl)tetrahydro-2H-pyran-2-yl)oxy)methyl)tetrahydro-2H-pyran-2-yl)oxy)-6-hydroxy-6-methylheptan-2-yl)-11-hydroxy-4,4,9,13,14-pentamethyl-2,3,4,7,8,9,10,11,12,13,14,15,16,17-tetradecahydro-1H-cyclopenta[a]phenanthren-3-yl)oxy)-4,5-dihydroxy-2-(hydroxymethyl)tetrahydro-2H-pyran-3-yl)oxy)-6-(hydroxymethyl)tetrahydro-2H-pyran-3,4,5-triol, or by the abbreviation "MC1".

The sugar moieties shown in the structure above are shown in their closed, cyclic form. The structure also encompasses forms where the depicted sugar moieties are in their open, acyclic form.

Isotopes may be present in the compounds described. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise.

In some embodiments, the MC1 compound capable of forming acid or base salts by virtue of the presence of amino or carboxyl groups or groups similar thereto. Comestibly acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Comestibly acceptable salts can be formed using inorganic and organic bases. Inorganic bases from which salts can be derived include, for example, bases that contain sodium, potassium, lithium, ammonium, calcium, magnesium, iron, zinc, copper, manganese, aluminum, and the like; particularly preferred are the ammonium, potassium, sodium, calcium and magnesium salts. In some embodiments, treatment of the compounds disclosed herein with an inorganic base results in loss of a labile hydrogen from the compound to afford the salt form including an inorganic cation such as Li⁺, Na⁺, K⁺, Mg²⁺ and Ca²⁺ and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. In some embodiments, the salts are comestibly acceptable salts, which are salts suitable for inclusion in ingestible compositions, such as food or beverage products.

In some embodiments, the sweetening compound is the MC1 compound in its free (non-salt) form. In other embodiments, the sweetening compound is a comestibly acceptable salt of the MC1 compound.

In some embodiments, the MC1 compound, or its comestibly acceptable salts, exist as a crystalline solid, either in substantially pure form or in a formulation such as those set forth below. The crystalline solid can have any suitable polymorphic form, such as any polymorphic form obtainable via recrystallization in any suitable solvent system, according to techniques commonly used in the art of polymorph screening.

In some other embodiments, the MC1 compound, or its comestibly acceptable salts, exist as an amorphous solid or a semi-amorphous solid, meaning that it lacks any regular crystalline structure. Such solids can be generated using standard techniques, such as spray drying, and the like.

In some embodiments, the MC1 compound, or its comestibly acceptable salts, exist as a solvate, which is a pseudomorphic form of the compound in which one or more solvent molecules (such as water molecules) are taken up into the crystalline structure. Any suitable solvent or combination of solvents can be used, including, but not limited to, water, methanol, ethanol, n-propanol, isopropanol, n-butanol, 2-butanol, isobutanol, ethyl acetate, ethylene glycol, 1,2-propylene glycol, 1,3-propylene glycol, and the like. In some embodiments, the disclosure provides hydrates of the MC1 compound. Such solvates can be generated by any suitable means, such as those techniques typically used by skilled artisans in the field of polymorph and solvate screening.

In some other embodiments, the MC1 compound, or its comestibly acceptable salts, exist as a co-crystal with one or more other compounds, such as one or more other sweetener compounds. The MC1 compound, or its comestibly acceptable salts, can form a co-crystal with any suitable compound. Non-limiting examples of such suitable compounds include fructose, glucose, galactose, sucrose, lactose, maltose, allulose, sugar alcohols (such as erythritol, sorbitol, xylitol, and the like), sucralose, steviol glycosides (such as rebaudioside A, rebaudioside B, rebaudioside D, rebaudioside E, rebaudioside M, and the like natural stevioside compounds), other mogrosides (such as mogroside V, isomogroside V, siamenoside I, isomogroside III_{E}, the 1,6-α isomer of siamenoside I, and the like), aspartame, saccharin, acesulfame K, cyclamate, inulin, isomalt, and maltitol. Such co-crystals can be generated by any suitable means, such as those set forth in U.S. Patent Application Publication No. 2018/0363074.

In some embodiments, the MC1 compound, or its comestibly acceptable salts, are in the form of a dry particle. Such dry particles can be formed by standard techniques in the art, such as dry granulation, wet granulation, and the like. Such particles can also contain a number of excipients, including, inert diluents, such as calcium carbonate, sodium carbonate, lactose, calcium phosphate, and sodium phosphate; granulating and disintegrating agents, such as starch, cellulosic materials, and alginic acid; binding agents, such as gelatin, guar gum, and acacia; and lubricating agents, such as magnesium stearate, stearic acid, and talc. Other excipients typically used in food and beverage products can also be included, such as typical foodstuff materials.

In some embodiments, the MC1 compound, or its comestibly acceptable salts, are in the form of a liquid solution or a liquid suspension. Such compositions can also include: carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. Such compositions can also include one or more coloring agents, one or more flavoring agents, and the like. Such liquid suspensions and solutions have a liquid carrier. In general, the liquid carrier comprises water. In some such cases, the liquid composition is an emulsion, such as an oil-in-water or a water-in-oil emulsion. Further, in some cases, water may be too polar to dissolve the MC1 compound to the desired concentration. In such instances, it can be desirable to introduce water-miscible solvents, such as alcohols, glycols, polyols, and the like, to the solvent to enhance solubilization of the MC1 compound.

In some embodiments, the MC1 compound, or its comestibly acceptable salts, are in the form of a solution, i.e., are solvated within a liquid carrier. In some embodiments, the liquid carrier is an aqueous carrier. Such solutions can be diluted to any suitable concentration.

### Uses and Methods

In certain aspects, the disclosure provides uses of the MC1 compound, or its comestible acceptable salts, to modify the flavor of or to impart a flavor to, such as to impart sweetness to (i.e., sweeten), an ingestible composition. The disclosure provides related methods of modifying a flavor of or imparting a flavor to, such as to impart sweetness to, an ingestible composition, the method comprising introducing a sweetening compound of the first aspect to an ingestible composition. In some embodiments, the sweetening compounds are used in or introduced to the ingestible composition in a concentration ranging from 0.01 ppm to 1000 ppm. In some embodiments, the ingestible composition comprises a juice of an extract of monk fruit.

In certain aspects, the disclosure provides uses of the MC1 compound, or its comestible acceptable salts, to reduce the caloric content of an ingestible composition. The disclosure provides related methods of reducing the caloric content of an ingestible composition, the method comprising introducing a sweetening compound of the first aspect to an ingestible composition. In some embodiments, such calorie reduction occurs by permitting the reduced use of caloric sweeteners, such as sucrose, fructose, or glucose. In some embodiments, the sweetening compounds are used in or introduced to the ingestible composition in a concentration ranging from 0.01 ppm to 1000 ppm. In some embodiments, the ingestible composition comprises a juice of an extract of monk fruit.

The preceding uses and methods involve ingestible compositions. In addition to the features of those ingestible compositions set forth above, the ingestible compositions can incorporate any features or combinations of features set forth below.

### Ingestible Compositions

In certain aspects, the disclosure provides ingestible compositions comprising the MC1 compound, or its comestibly acceptable salts, according to the embodiments set forth above. When introduced to or used in the ingestible composition, the MC1 compound, or its comestibly acceptable salts, are used or introduced to in the ingestible composition at a concentration ranging from 0.01 ppm to 1000 ppm, or from 0.01 ppm to 900 ppm, or from 0.01 ppm to 800 ppm, or from 0.01 ppm to 700 ppm, or from 0.01 ppm to 600 ppm, or from 0.1 ppm to 500 ppm, or from 0.1 ppm to 400 ppm, or from 0.1 ppm to 300 ppm, or from 0.1 ppm to 200 ppm, or from 1 ppm to 1000 ppm, or from 1 ppm to 900 ppm, or from 1 ppm to 800 ppm, or from 1 ppm to 700 ppm, or from 1 ppm to 600 ppm, or from 1 ppm to 500 ppm, or from 50 ppm to 300 ppm.

In some instances, the ingestible composition represents a substantially pure and isolated composition of the MC1 compound. Thus, in some embodiments, the disclosure provides a ingestible composition comprising at least 50% by weight, or at least 60% by weight, or at least 70% by weight, or at least 80% by weight, or at least 90% by weight, or at least 95% by weight, or at least 97% by weight, or at least 99% by weight, of the MC1 compound, based on the total dry weight of the composition.

In some embodiments, the ingestible composition comprises one or more bitter tastants. In some embodiments, the bitter tastant is a high-intensity sweetener, such as acesulfame potassium, aspartame, neotame, cyclamate, saccharin, sucralose, steviol glycodises (such as rebaudioside A, rebaudioside B, rebaudioside M, rebaudioside D, or rebaudioside E), and mogrosides (such as mogroside III, mogroside IV, mogroside V, siamenoside I, isomogroside V, mogroside IV_{E}, isomogroside IV, mogroside III_{E}, 11-oxomogroside V, or the 1,6-α isomer of siamenoside I). Thus, the MC1 compound, or its comestibly acceptable salts, may be suitable used in reduced-sugar or zero-sugar products to offset the bitterness or lingering aftertaste (e.g., licorice aftertaste) imparted by the use of such low-calorie or zero-calorie sweeteners.

In some embodiments, the bitter tastant is a potassium salt, such as potassium chloride, which is often used as a partial or complete replacement of sodium chloride in certain low-sodium or zero-sodium foods. Thus, the MC1 compound, or its comestibly acceptable salts, may be suitable used in such products to offset the bitterness imparted by potassium salts.

In some embodiments, the bitter tastant a non-animal protein, such as a plant protein, an algal protein, or a mycoprotein. In some embodiments, the ingestible composition comprises a plant protein. Non-limiting examples of plant proteins include pea protein, soy protein, almond protein, cashew protein, canola (rapeseed) protein, chickpea protein, fava protein, sunflower protein, wheat protein, oat protein, barley protein, and potato protein. Such non-animal proteins are often used as a partial or full replacement of animal proteins in dairy analogues and meat analogues. Thus, the MC1 compound, or its comestibly acceptable salts, may be suitably used in such products to offset the bitterness imparted by the non-animal proteins. By offsetting the bitterness of such proteins, the MC1 compound, or its comestibly acceptable salts, can reduce the perceived cereal notes and green notes experienced by consumers.

In some embodiments, the bitter tastant is caffeine, quinine, green tea, catechins, polyphenols (such as a polyphenol antioxidants), tannins, green robusta coffee extract, green coffee extract, menthol, and the like. Such compounds commonly occur in various natural foods products, such as tea and coffee, and in packaged food products, such as instant tea, instant coffee, packaged beverages, and the like. When one or more such bitter tastants are present, the MC1 compound, or its comestibly acceptable salts, can be suitably used to offset the bitterness of such compounds and improve the perceived taste of the product to consumers.

In some embodiments, the bitter tastant is a pharmaceutical compound. Non-limiting examples of pharmaceutical compounds having a bitter taste include atropine, brinzolamide, chloramphenicol, chloroquine, clindamycin, dexamethasone, digoxin, diltiazem, diphenhydramine, docusate, dorzolamide, doxepin, doxylamine, enalapril, erythromycin, esomeprazole, famotidine, gabapentin, ginkgolide A, guaifenesin, L-histidine, lomefloxacin, methylprednisolone, ofloxacin, oleuropein, oxyphenonium, pirenzepine, prednisone, ranitidine, trapidil, trimethoprim, and cetirizine. When one or more such pharmaceutical compounds are used in an oral pharmaceutical formulation, the MC1 compound, or its comestibly acceptable salts, can be suitably used to offset the bitterness of such compounds and improve the perceived taste of the pharmaceutical product to consumers.

In some embodiments, the bitter tastant is an oral care ingredient. Many oral care ingredients impart a bitter off taste, which must be masked or blocked to improve consumer acceptance of the product. Non-limiting examples of such oral care ingredients include menthol, menthol analogues, mint extracts, sodium bicarbonate, alkali metal salts of peroxymonosulfate (potassium peroxymonosulfate), cetylpyridinium chloride, lauramidopropyl betaine, cocamidopropyl betaine, arginine, hydrogen peroxide, chlorhexidine gluconate, potassium nitrate, pentasodium triphosphate, tetrasodium pyrophosphate, stannous fluoride, thymol, methyl salicylate, eucalyptol, thymol, cubebol, and any combination thereof. When one or more such oral care compounds are used in oral care products, the MC1 compound, or its comestibly acceptable salts, can be suitably used to offset the bitterness of such compounds and improve the perceived taste of the oral care product to consumers.

In some embodiments, the bitter tastant is a bitter agent found in citrus, such as limonin, nomelin, or naringin. Many citrus-containing preparatuions impart a bitter off taste, which must be masked or blocked to improve consumer acceptance of the product. This bitter off-taste can, in some cases, be attributed to the citrus greening disease, which cases citrus fruits to turn green before fully ripening. When one or more such citrus bitter agents are present in a product the MC1 compound, or its comestibly acceptable salts, can be suitably used to offset the bitterness of such compounds and improve the perceived taste of the citrus product to consumers.

In some embodiments, the ingestible composition comprises the juice or extract of monk fruit. The term "monk fruit" refers to the fruit of the plant *Siraitia grosvenorii,* which is also called "luo han guo" or "LHG." The term "monk fruit juice" refers to a composition obtained by crushing the fruit and removing at least a portion of its fibrous material. The term "monk fruit extract" refers to the composition obtained from subjecting the monk fruit juice to any suitable process to increase the concentration of mogroside compounds, such as mogroside V, in the composition relative to their concentration in the juice. Such mogroside juices and extracts can be subjected to decolorization by any suitable means. In embodiments that comprise monk fruit juice or monk fruit extract, the MC1 compound, or its comestibly acceptable salts, can be present any suitable ratio relative to mogroside V. For example, in some such embodiments, the ingestible composition comprises the MC1 compound, or its comestibly acceptable salts, in a weight ratio relative to mogroside V in an amount ranging from 1:100 to 100:1, or from 1:50 to 50:1, or from 1:25 to 25:1, or from 1:10 to 10:1, or from 1:5 to 5:1, or from 1:3 to 3:1, or from 1:2 to 2:1.

In some embodiments, the ingestible composition includes other sweeteners or combinations of other sweeteners. In some embodiments, the other sweetener is a common saccharide sweetener, such as sucrose, fructose, glucose, and sweetener compositions comprising natural sugars, such as corn syrup (including high fructose corn syrup) or other syrups or sweetener concentrates derived from natural fruit and vegetable sources. In some embodiments, the sweetener is sucrose, fructose, or a combination thereof. In some embodiments, the sweetener is sucrose. In some other embodiments, the sweetener is selected from rare natural sugars including D-allose, D-psicose, L-ribose, D-tagatose, L-glucose, L-fucose, L-arbinose, D-turanose, and D-leucrose. In some embodiments, the sweetener is selected from semi-synthetic "sugar alcohol" sweeteners such as erythritol, isomalt, lactitol, mannitol, sorbitol, xylitol, maltodextrin, and the like. In some embodiments, the sweetener is selected from artificial sweeteners such as aspartame, saccharin, acesulfame-K, cyclamate, sucralose, and alitame. In some embodiments, the sweetener is selected from the group consisting of cyclamic acid, mogroside, tagatose, maltose, galactose, mannose, sucrose, fructose, lactose, allulose, neotame and other aspartame derivatives, glucose, D-tryptophan, glycine, maltitol, lactitol, isomalt, hydrogenated glucose syrup (HGS), hydrogenated starch hydrolyzate (HSH), stevioside, rebaudioside A, other sweet Stevia-based glycosides, chemically modified steviol glycosides (such as glucosylated steviol glycosides), mogrosides, chemically modified mogrosides (such as glucosylated mogrosides), carrelame and other guanidine-based sweeteners. In some embodiments, the sweetener is a combination of two or more of the sweeteners set forth in this paragraph. In some embodiments, the sweetener may combinations of two, three, four or five sweeteners as disclosed herein. In some embodiments, the sweetener may be a sugar. In some embodiments, the sweetener may be a combination of one or more sugars and other natural and artificial sweeteners. In some embodiments, the sweetener is a sugar. In some embodiments, the sugar is cane sugar. In some embodiments, the sugar is beet sugar. In some embodiments, the sugar may be sucrose, fructose, glucose or combinations thereof. In some embodiments, the sugar may be sucrose. In some embodiments, the sugar may be a combination of fructose and glucose.

The other sweetener can also include, for example, sweetener compositions comprising one or more natural or synthetic carbohydrate, such as corn syrup, high fructose corn syrup, high maltose corn syrup, glucose syrup, sucralose syrup, hydrogenated glucose syrup (HGS), hydrogenated starch hydrolyzate (HSH), or other syrups or sweetener concentrates derived from natural fruit and vegetable sources, or semi-synthetic "sugar alcohol" sweeteners such as polyols. Non-limiting examples of polyols in some embodiments include erythritol, maltitol, mannitol, sorbitol, lactitol, xylitol, isomalt, propylene glycol, glycerol (glycerin), threitol, galactitol, palatinose, reduced isomalto-oligosaccharides, reduced xylo-oligosaccharides, reduced gentio-oligosaccharides, reduced maltose syrup, reduced glucose syrup, isomaltulose, maltodextrin, and the like, and sugar alcohols or any other carbohydrates or combinations thereof capable of being reduced which do not adversely affect taste.

The other sweetener may be a natural or synthetic sweetener that includes, but is not limited to, agave inulin, agave nectar, agave syrup, amazake, brazzein, brown rice syrup, coconut crystals, coconut sugars, coconut syrup, date sugar, fructans (also referred to as inulin fiber, fructo-oligosaccharides, or oligo-fructose), green stevia powder, stevia rebaudiana, rebaudioside A, rebaudioside B, rebaudioside C, rebaudioside D, rebaudioside E, rebaudioside F, rebaudioside I, rebaudioside H, rebaudioside L, rebaudioside K, rebaudioside J, rebaudioside N, rebaudioside O, rebaudioside M and other sweet stevia-based glycosides, stevioside, stevioside extracts, honey, Jerusalem artichoke syrup, licorice root, luo han guo (fruit, powder, or extracts), lucuma (fruit, powder, or extracts), maple sap (including, for example, sap extracted *from Acer saccharum, Acer nigrum, Acer rubrum, Acer saccharinum, Acer platanoides, Acer negundo, Acer macrophyllum, Acer grandidentatum, Acer glabrum, Acer mono*), maple syrup, maple sugar, walnut sap (including, for example, sap extracted from *Juglans cinerea, Juglans nigra, Juglans ailatifolia, Juglans regia*), birch sap (including, for example, sap extracted from *Betula papyrifera, Betula alleghaniensis, Betula lenta, Betula nigra, Betula populifolia, Betula pendula*)*,* sycamore sap (such as, for example, sap extracted from *Platanus occidentalis*), ironwood sap (such as, for example, sap extracted from *Ostrya virginiana*), mascobado, molasses (such as, for example, blackstrap molasses), molasses sugar, monatin, monellin, cane sugar (also referred to as natural sugar, unrefined cane sugar, or sucrose), palm sugar, panocha, piloncillo, rapadura, raw sugar, rice syrup, sorghum, sorghum syrup, cassava syrup (also referred to as tapioca syrup), thaumatin, yacon root, malt syrup, barley malt syrup, barley malt powder, beet sugar, cane sugar, crystalline juice crystals, caramel, carbitol, carob syrup, castor sugar, hydrogenated starch hydrolates, hydrolyzed can juice, hydrolyzed starch, invert sugar, anethole, arabinogalactan, arrope, syrup, P-4000, acesulfame potassium (also referred to as acesulfame K or ace-K), alitame (also referred to as aclame), advantame, aspartame, baiyunoside, neotame, benzamide derivatives, bernadame, canderel, carrelame and other guanidine-based sweeteners, vegetable fiber, corn sugar, coupling sugars, curculin, cyclamates, cyclocarioside I, demerara, dextran, dextrin, diastatic malt, dulcin, sucrol, valzin, dulcoside A, dulcoside B, emulin, enoxolone, maltodextrin, saccharin, estragole, ethyl maltol, glucin, gluconic acid, glucono-lactone, glucosamine, glucoronic acid, glycerol, glycine, glycyphillin, glycyrrhizin, glycyrrhetic acid monoglucuronide, golden sugar, yellow sugar, golden syrup, granulated sugar, gynostemma, hernandulcin, isomerized liquid sugars, jallab, chicory root dietary fiber, kynurenine derivatives (including N'-formyl-kynurenine, N'-acetyl-kynurenine, 6-chloro-kynurenine), galactitol, litesse, ligicane, lycasin, lugduname, guanidine, falemum, mabinlin I, mabinlin II, maltol, maltisorb, maltodextrin, maltotriol, mannosamine, miraculin, mizuame, mogrosides (including, for example, mogroside IV, mogroside V, and neomogroside), mukurozioside, nano sugar, naringin dihydrochalcone, neohesperidine dihydrochalcone, nib sugar, nigero-oligosaccharide, norbu, orgeat syrup, osladin, pekmez, pentadin, periandrin I, perillaldehyde, perillartine, petphyllum, phenylalanine, phlomisoside I, phlorodizin, phyllodulcin, polyglycitol syrups, polypodoside A, pterocaryoside A, pterocaryoside B, rebiana, refiners syrup, rub syrup, rubusoside, selligueain A, shugr, siamenoside I, siraitia grosvenorii, soybean oligosaccharide, Splenda, SRI oxime V, steviol glycoside, steviolbioside, stevioside, strogins 1, 2, and 4, sucronic acid, sucrononate, sugar, suosan, phloridzin, superaspartame, tetrasaccharide, threitol, treacle, trilobtain, tryptophan and derivatives (6-trifluoromethyl-tryptophan, 6-chloro-D-tryptophan), vanilla sugar, volemitol, birch syrup, aspartame-acesulfame, assugrin, and combinations or blends of any two or more thereof.

Other sweeteners also include combinations of any two or more of any of the aforementioned sweeteners. In some embodiments, the sweetener may comprise combinations of two, three, four or five sweeteners as disclosed herein. In some embodiments, the sweetener may be a sugar. In some embodiments, the sweetener may be a combination of one or more sugars and other natural and artificial sweeteners. In some embodiments, the sweetener is a caloric sweetener, such as sucrose, fructose, xylitol, erythritol, or combinations thereof. In some embodiments, the ingestible compositions are free (or, in some embodiments) substantially free of stevia-derived sweeteners, such as steviol glycosides, glucosylated steviol glycosides, or rebaudiosides.

In some embodiments, the sweetener is sucrose, fructose, glucose, or any combination thereof. In some other embodiments, the sweetener is a steviol glycoside, such as rebaudioside A, rebaudioside M, and the like.

In some embodiments, the ingestible composition comprises a sour tastant, such as an organic acid. Non-limiting examples of such organic acids include acetic acid, malonic acid, citric acid, lactic acid, and the like.

The ingestible compositions can, in certain embodiments, comprise any additional ingredients or combination of ingredients as are commonly used in food and beverage products, including, but not limited to:
acids, including, for example citric acid, phosphoric acid, ascorbic acid, sodium acid sulfate, lactic acid, or tartaric acid;
bitter ingredients, including, for example caffeine, quinine, green tea, catechins, polyphenols, green robusta coffee extract, green coffee extract, potassium chloride, menthol, or proteins (such as proteins and protein isolates derived from plants, algae, or fungi);
coloring agents, including, for example caramel color, Red #40, Yellow #5, Yellow #6, Blue #1, Red #3, purple carrot, black carrot juice, purple sweet potato, vegetable juice, fruit juice, beta carotene, turmeric curcumin, or titanium dioxide;
preservatives, including, for example sodium benzoate, potassium benzoate, potassium sorbate, sodium metabisulfate, sorbic acid, or benzoic acid;
antioxidants including, for example ascorbic acid, calcium disodium EDTA, alpha tocopherols, mixed tocopherols, rosemary extract, grape seed extract, resveratrol, or sodium hexametaphosphate;
vitamins or functional ingredients including, for example resveratrol, Co-Q10, omega 3 fatty acids, theanine, choline chloride (citocoline), fibersol, inulin (chicory root), taurine, panax ginseng extract, guanana extract, ginger extract, L-phenylalanine, L-camitine, L-tartrate, D-glucoronolactone, inositol, bioflavonoids, Echinacea, ginko biloba, yerba mate, flax seed oil, garcinia cambogia rind extract, white tea extract, ribose, milk thistle extract, grape seed extract, pyrodixine HCl (vitamin B6), cyanoobalamin (vitamin B12), niacinamide (vitamin B3), biotin, calcium lactate, calcium pantothenate (pantothenic acid), calcium phosphate, calcium carbonate, chromium chloride, chromium polynicotinate, cupric sulfate, folic acid, ferric pyrophosphate, iron, magnesium lactate, magnesium carbonate, magnesium sulfate, monopotassium phosphate, monosodium phosphate, phosphorus, potassium iodide, potassium phosphate, riboflavin, sodium sulfate, sodium gluconate, sodium polyphosphate, sodium bicarbonate, thiamine mononitrate, vitamin D3, vitamin A palmitate, zinc gluconate, zinc lactate, or zinc sulfate;
clouding agents, including, for example ester gun, brominated vegetable oil (BVO), or sucrose acetate isobutyrate (SAIB);
buffers, including, for example sodium citrate, potassium citrate, or salt;
flavors, including, for example propylene glycol, ethyl alcohol, glycerine, gum Arabic (gum acacia), maltodextrin, modified corn starch, dextrose, natural flavor, natural flavor with other natural flavors (natural flavor WONF), natural and artificial flavors, artificial flavor, silicon dioxide, magnesium carbonate, or tricalcium phosphate; or
starches and stabilizers, including, for example pectin, xanthan gum, carboxylmethylcellulose (CMC), polysorbate 60, polysorbate 80, medium chain triglycerides, cellulose gel, cellulose gum, sodium caseinate, modified food starch, gum Arabic (gum acacia), inulin, or carrageenan.

The ingestible compositions can have any suitable pH. In some embodiments, the MC1 compound, or its comestibly acceptable salts, enhance the sweetness of a sweetener under a broad range of pH, e.g., from lower pH to neutral pH. The lower and neutral pH includes, but is not limited to, a pH from 1.5 to 9.0, or from 2.5 to 8.5; from 3.0 to 8.0; from 3.5 to 7.5; and from 4.0 to 7. In certain embodiments, compounds as disclosed and described herein, individually or in combination, can enhance the perceived sweetness of a fixed concentration of a sweetener in taste tests at a compound concentration of 50 µM, 40 µM, 30 µM, 20 µM, or 10 µM at both low to neutral pH value. In certain embodiments, the enhancement factor of the compounds as disclosed and described herein, individually or in combination, at the lower pH is substantially similar to the enhancement factor of the compounds at neutral pH. Such consistent sweet enhancing property under a broad range of pH allow a broad use in a wide variety of foods and beverages of the compounds as disclosed and described herein, individually or in combination.

In some embodiments, the ingestible composition comprises a flavoring. Any suitable flavoring can be used. In some embodiments, the flavoring comprises synthetic flavor oils and flavoring aromatics or oils, oleoresins and extracts derived from plants, leaves, flowers, fruits, and so forth, or combinations thereof. Non-limiting examples of flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Non-limiting examples of other flavors include natural and synthetic fruit flavors such as vanilla, and citrus oils including lemon, orange, lime, grapefruit, yazu, sudachi, and fruit essences including apple, pear, peach, grape, blueberry, strawberry, raspberry, cherry, plum, pineapple, watermelon, apricot, banana, melon, apricot, ume, cherry, raspberry, blackberry, tropical fruit, mango, mangosteen, pomegranate, papaya and so forth. Other potential flavors include a milk flavor, a butter flavor, a cheese flavor, a cream flavor, and a yogurt flavor; a vanilla flavor; tea or coffee flavors, such as a green tea flavor, a oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a chamomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. In the context of dairy or dairy analog products, the most commonly used flavor agents are agents that impart flavors such as vanilla, French vanilla, chocolate, banana, lemon, hazelnut, coconut, almond, strawberry, mocha, coffee, tea, chai, cinnamon, caramel, cream, brown sugar, toffee, pecan, butter pecan, toffee, Irish crème, white chocolate, raspberry, pumpkin pie spice, peppermint, or any combination thereof.

In some embodiments, the ingestible composition comprises vanillin or a vanillin analogue, which impart a vanilla flavor to the flavoring. In some further embodiments, the ingestible composition comprises one or more lactones, which impart a creamy flavor to the composition.

In some embodiments, the ingestible composition comprises a yeast extract, such as a yeast lysate. Such extracts can be obtained from any suitable yeast strain, where such extracts are suitable for human consumption. Non-limiting examples of such yeasts include: yeasts of the genus *Saccharomyces,* such as *Saccharomyces cerevisiae* or *Saccharomyces pastorianus;* yeasts of the genus *Candida,* such as *Candida utilis;* yeasts of the genus *Kluyveromyces,* such as *Kluyveromyces lactis* or *Kluyveromyces marxianus;* yeasts of the genus *Pichia* such as *Pichia pastoris;* yeasts of the genus *Debaryomyces* such as *Debaryomyces hansenii;* and yeasts of the genus *Zygosaccharomyces* such as *Zygosaccharomyces mellis.* In some embodiments, the yeast is a yeast collected after brewing beer, sake, or the like. In some embodiments, the yeast is a yeast subjected to drying treatment (dried yeast) after collection.

Such extracts can be produced by any suitable means. In general, yeast extracts or lysates are made by extracting the contents of the yeast cells from the cell wall material. In many instances, the digestive enzymes in the cells (or additional enzymes added to the composition) break down the proteins and polynucleotides in the yeast to amino acids, oligopeptides (for example, from 2 to 10 peptides), nucleotides, oligonucleotides (from 2 to 10 nucleotides), and mixtures thereof. A yeast lysate can be prepared by lysing a yeast. For example, in some embodiments, the yeast after culture is crushed or lysed by an enzymatic decomposition method, a self-digestion method, an alkaline extraction method, a hot water extraction method, an acid decomposition method, an ultrasonic crushing method, crushing with a homogenizer, a freezing-thawing method, or the like (two or more thereof may be used in combination), whereby a yeast lysate is obtained. Yeast may be cultured by a conventional method. In some embodiments, the yeast after culture is heat-treated and then treated with a lytic enzyme to obtain an enzyme lysate. The conditions for the heat treatment are, for example, 80°C to 90 °C for 5 minutes to 30 minutes. As the lytic enzyme used for the enzymatic decomposition method, various enzymes can be used as long as they can lyse the cell wall of yeast. The reaction conditions may be set so as to be optimum or suitable for the lytic enzyme(s) to be used, and specific examples thereof can include a temperature of 50°C to 60 °C, and a pH of 7.0 to 8.0. The reaction time is also not particularly limited, and can be, for example, 3 hours to 5 hours.

Compositions comprising yeast lysate can be obtained from a variety of commercial sources. For example, in some embodiments, the yeast lysate is provides by the flavoring additive sold under the name MODUMAX (DSM Food Specialties BV, Delft, Netherlands).

In some embodiments, the ingestible composition comprises a sweetness enhancer. Any suitable sweetness enhancer can be used in the ingestible compositions disclosed herein, including synthetic sweetness enhancers, natural sweetness enhancers, or any combinations thereof.

Examples of suitable synthetic sweetness enhancers include, but are not limited to, *N*-(1-((4-amino-2,2-dioxo-1*H*-benzo[c][1,2,6]thiadiazin-5-yl)oxy)-2-methylpropan-2-yl)isonicotinamide, or any of its comestbly acceptable salts, 3-hydroxybenzoic acid, or any compounds set forth in U.S. Patent Nos. 8,541,421; 8,815,956; 9,834,544; 8,592,592; 8,877,922; 9,000,054; and 9,000,051, as well as U.S. Patent Application Publication No. 2017/0119032.

Suitable examples of natural sweetness enhancers include, but are not limited to, hesperetin dihydrochalcone, hesperetin dihydrochalcone-4'-O'glucoside, neohesperetin dihydrochalcone, brazzein, hesperidin, phyllodulcin, naringenin, naringin, phloretin, glucosylated steviol glycosides, (2R,3R)-3-acetoxy-5,7,4'-trihydroxyflavanone, (2R,3R)-3-acetoxy-5,7,3'-trihydroxy-4'-methoxyflavanone, rubusosides, thaumatin, monellin, miraculin, glycyrrhizin and comestible acceptable salts thereof (such as the monoammonium salt), naringin dihydrochalcone, myricetin, nobiletin, polymethoxyflavones, mixed methoxy- and hydroxyflavones, quercetin, certain amino acids, and the like. As used herein, the term "glucosylated steviol glycoside" refers to the product of enzymatically glucosylating natural steviol glycoside compounds. The glucosylation generally occurs through a glycosidic bond, such as an α-1,2 bond, an α-1,4 bond, an α-1.6 bond, a β-1,2 bond, a β-1,4 bond, a β-1,6 bond, and so forth.

In some embodiments of any of the preceding embodiments, the ingestible composition comprises 3-((4-amino-2,2-dioxo-1*H*-benzo[*c*][1,2,6]thiadiazin-5-yl)oxy)-2,2-dimethyl-*N*-propyl-propanamide, *N*-(1-((4-amino-2,2-dioxo-1*H*-benzo[c][1,2,6]-thiadiazin-5-yl)oxy)-2-methyl-propan-2-yl)isonicotinamide, or a comestibly acceptable salt thereof. In some embodiments, the ingestible composition comprises *N*-(1-((4-amino-2,2-dioxo-1*H*-benzo[*c*][1,2,6]thiadiazin-5-yl)oxy)-2-methyl-propan-2-yl)isonicotinamide, or a comestbly acceptable salt thereof. In some embodiments, the ingestible composition comprises *N*-(1-((4-amino-2,2-dioxo-1*H*-benzo[c][1,2,6]thiadiazin-5-yl)oxy)-2-methyl-propan-2-yl)isonicotinamide.

In some embodiments, the ingestible composition comprises one or more umami enhancing compounds. Such umami enhancing compounds include, but are not limited to, naturally derived compounds, or synthetic compounds, such as any compounds set forth in U.S. Patent Nos. 8,735,081; 8,124,121; and 8,968,708. In some embodiments, the umami-enhancing compound is (2R,4R)-1,2,4-trihydroxy-heptadec-16-ene, (2R,4R)-1,2,4-trihydroxyheptadec-16-yne, or a mixture thereof. In some embodiments, the umami-enhancing compound is (3R,5S)-1-(4-hydroxy-3-methoxyphenyl)decane-3,5-diol diacetate. In some embodiments, the umami-enhancing compound is N-(heptan-4-yl)benzo-[d] [1,3] dioxole-5-carboxamide.

In some further embodiments, the ingestible composition comprises one or more cooling enhancing compounds. Such cooling enhancing compounds include, but are not limited to, naturally derived compounds, such as menthol or analogs thereof, or synthetic compounds, such as any compounds set forth in U.S. Patent Nos. 9,394,287 and 10,421,727. Non-limiting examples include N-ethyl-N-(thiophen-2-ylmethyl)-2-(p-tolyloxy)acetamide, N-(1H-pyrazol-3-yl)-N-(thiophen-2-ylmethyl)-2-(p-tolyloxy)acetamide, 2-(4-fluorophenoxy)-N-(1H-pyrazol-3-yl)-N-(thiophen-2-ylmethyl)acetamide, 2-(2-hydroxy-4-methylphenoxy)-N-(1H-pyrazol-3-yl)-N-(thiophen-2-ylmethyl)-acetamide, 2-((2,3-dihydro-1H-inden-5-yl)oxy)-N-(1H-pyrazol-3-yl)-N-(thiophen-2-ylmethyl)-acetamide, 2-((2,3-dihydro-1H-inden-5-yl)oxy)-N-(1H-pyrazol-3-yl)-N-(thiazol-5-ylmethyl)-acetamide, 2-((5-methoxybenzofuran-2-yl)oxy)-N-(1H-pyrazol-3-yl)-N-(thiophen-2-ylmethyl)-acetamide, (E/Z)-2-methyl-2-butenal, (E/Z)-2-isopropyl-5-methyl-2-hexenal, phloretin, naringenin, and any combinations thereof.

In some further embodiments, the ingestible composition comprises one or more bitterness blocking or bitter masking compounds. Such bitterness blocking compounds or bitter masking include, but are not limited to, naturally derived compounds or synthetic compounds, such as any compounds set forth in U.S. Patent Nos. 8,076,491; 8,445,692; and 9,247,759. Non-limiting examples include 3-(1-((3,5-dimethylisoxazol-4-yl)-methyl)-1*H*-pyrazol-4-yl)-1-(3-hydroxybenzyl)-imidazolidine-2,4-dione, 4-(2,2,3-trimethyl-cyclopentyl)butanoic acid, 3β-hydroxydihydrocostunolide, 3β-hydroxypelenolide, probenecid, sakuranetin, 6-methoxysakuranetin, jaceosidin, 4'-fluoro-6-methoxyflavonone, 6,3'-dimethoxyflavonone, 6-methoxyflavonone, γ-aminobutyric acid, Nα,Nα-bis(carbomethyl)-L-lysine, (+/-) abscisic acid, sodium gluconate, monosodium glutamate, sodium acetate, homoeriodictyol, sterubin, eriodictyol, 2,4,dihydrobenzoic acid, neodiosmin, 1-carboxymethyl-5-hydroxy-2-hydroxymethylpyridinium, flavan-3-spiro-C-glycosides, poly -γ-glutarmic acid, α,α-trehalose, taurine, (2)-gingerdione, 2,4,-dihydroxybenzoic acid, L-theanine, enterodiol, lariciresinol, enterolactone, matairesinol, and any combinations thereof.

In some further embodiments, the ingestible composition comprises one or more sour taste modulating compounds.

In some further embodiments, the ingestible composition comprises one or more mouthfeel modifying compounds. Such mouthfeel modifying compounds include, but are not limited to, tannins, cellulosic materials, bamboo powder, and the like.

In some further embodiments, the ingestible composition comprises one or more flavor masking compounds. Such flavor masking compounds include, but are not limited to, cellulosic materials, materials extracted from fungus, materials extracted from plants, citric acid, carbonic acid (or carbonates), and the like.

### Flavored Products

In certain aspects, the disclosure provides flavored products comprising any ingestible compositions of the preceding aspects. In some embodiments, the flavored products are beverage products, such as soda, flavored water, tea, and the like. In some other embodiments, the flavored products are food products, such as yogurt. In some embodiments, the flavored products are oral care products, such as toothpaste, mouthwash, dentrifrices, whitening agents and the like.

In embodiments where the flavored product is a beverage, the beverage may be selected from the group consisting of enhanced sparkling beverages, colas, lemon-lime flavored sparkling beverages, orange flavored sparkling beverages, grape flavored sparkling beverages, strawberry flavored sparkling beverages, pineapple flavored sparkling beverages, ginger-ales, root beers, fruit juices, fruit-flavored juices, juice drinks, nectars, vegetable juices, vegetable-flavored juices, sports drinks, energy drinks, enhanced water drinks, enhanced water with vitamins, near water drinks, coconut waters, tea type drinks, coffees, cocoa drinks, beverages containing milk components, beverages containing cereal extracts and smoothies. In some embodiments, the beverage may be a soft drink.

In certain embodiments of any aspects and embodiments set forth herein that refer to an flavored product, the flavored product is a non-naturally-occurring product, such as a packaged food or beverage product.

Further non-limiting examples of food and beverage products or formulations include sweet coatings, frostings, or glazes for such products or any entity included in the Soup category, the Dried Processed Food category, the Beverage category, the Ready Meal category, the Canned or Preserved Food category, the Frozen Processed Food category, the Chilled Processed Food category, the Snack Food category, the Baked Goods category, the Confectionery category, the Dairy Product category, the Ice Cream category, the Meal Replacement category, the Pasta and Noodle category, and the Sauces, Dressings, Condiments category, the Baby Food category, and/or the Spreads category.

In general, the Soup category refers to canned/preserved, dehydrated, instant, chilled, UHT and frozen soup. For the purpose of this definition soup(s) means a food prepared from meat, poultry, fish, vegetables, grains, fruit and other ingredients, cooked in a liquid which may include visible pieces of some or all of these ingredients. It may be clear (as a broth) or thick (as a chowder), smooth, pureed or chunky, ready-to-serve, semi-condensed or condensed and may be served hot or cold, as a first course or as the main course of a meal or as a between meal snack (sipped like a beverage). Soup may be used as an ingredient for preparing other meal components and may range from broths (consommé) to sauces (cream or cheese-based soups).

The Dehydrated and Culinary Food Category usually means: (i) Cooking aid products such as: powders, granules, pastes, concentrated liquid products, including concentrated bouillon, bouillon and bouillon like products in pressed cubes, tablets or powder or granulated form, which are sold separately as a finished product or as an ingredient within a product, sauces and recipe mixes (regardless of technology); (ii) Meal solutions products such as: dehydrated and freeze dried soups, including dehydrated soup mixes, dehydrated instant soups, dehydrated ready-to-cook soups, dehydrated or ambient preparations of ready-made dishes, meals and single serve entrees including pasta, potato and rice dishes; and (iii) Meal embellishment products such as: condiments, marinades, salad dressings, salad toppings, dips, breading, batter mixes, shelf stable spreads, barbecue sauces, liquid recipe mixes, concentrates, sauces or sauce mixes, including recipe mixes for salad, sold as a finished product or as an ingredient within a product, whether dehydrated, liquid or frozen.

The Beverage category usually means beverages, beverage mixes and concentrates, including but not limited to, carbonated and non-carbonated beverages, alcoholic and non-alcoholic beverages, ready to drink beverages, liquid concentrate formulations for preparing beverages such as sodas, and dry powdered beverage precursor mixes. The Beverage category also includes the alcoholic drinks, the soft drinks, sports drinks, isotonic beverages, and hot drinks. The alcoholic drinks include, but are not limited to beer, cider/perry, FABs, wine, and spirits. The soft drinks include, but are not limited to carbonates, such as colas and non-cola carbonates; fruit juice, such as juice, nectars, juice drinks and fruit flavored drinks; bottled water, which includes sparkling water, spring water and purified/table water; functional drinks, which can be carbonated or still and include sport, energy or elixir drinks; concentrates, such as liquid and powder concentrates in ready to drink measure. The drinks, either hot or cold, include, but are not limited to coffee or ice coffee, such as fresh, instant, and combined coffee; tea or ice tea, such as black, green, white, oolong, and flavored tea; and other drinks including flavor-, malt- or plant-based powders, granules, blocks or tablets mixed with milk or water.

The Snack Food category generally refers to any food that can be a light informal meal including, but not limited to Sweet and savory snacks and snack bars. Examples of snack food include, but are not limited to fruit snacks, chips/crisps, extruded snacks, tortilla/corn chips, popcorn, pretzels, nuts and other sweet and savory snacks. Examples of snack bars include, but are not limited to granola/muesli bars, breakfast bars, energy bars, fruit bars and other snack bars.

The Baked Goods category generally refers to any edible product the process of preparing which involves exposure to heat or excessive sunlight. Examples of baked goods include, but are not limited to bread, buns, cookies, muffins, cereal, toaster pastries, pastries, waffles, tortillas, biscuits, pies, bagels, tarts, quiches, cake, any baked foods, and any combination thereof.

The Ice Cream category generally refers to frozen dessert containing cream and sugar and flavoring. Examples of ice cream include, but are not limited to: impulse ice cream; take-home ice cream; frozen yoghurt and artisanal ice cream; soy, oat, bean (e.g., red bean and mung bean), and rice-based ice creams.

The Confectionery category generally refers to edible product that is sweet to the taste. Examples of confectionery include, but are not limited to candies, gelatins, chocolate confectionery, sugar confectionery, gum, and the likes and any combination products.

The Meal Replacement category generally refers to any food intended to replace the normal meals, particularly for people having health or fitness concerns. Examples of meal replacement include, but are not limited to slimming products and convalescence products.

The Ready Meal category generally refers to any food that can be served as meal without extensive preparation or processing. The ready meal includes products that have had recipe "skills" added to them by the manufacturer, resulting in a high degree of readiness, completion and convenience. Examples of ready meal include, but are not limited to canned/preserved, frozen, dried, chilled ready meals; dinner mixes; frozen pizza; chilled pizza; and prepared salads.

The Pasta and Noodle category includes any pastas and/or noodles including, but not limited to canned, dried and chilled/fresh pasta; and plain, instant, chilled, frozen and snack noodles.

The Canned/Preserved Food category includes, but is not limited to canned/preserved meat and meat products, fish/seafood, vegetables, tomatoes, beans, fruit, ready meals, soup, pasta, and other canned/preserved foods.

The Frozen Processed Food category includes, but is not limited to frozen processed red meat, processed poultry, processed fish/seafood, processed vegetables, meat substitutes, processed potatoes, bakery products, desserts, ready meals, pizza, soup, noodles, and other frozen food.

The Dried Processed Food category includes, but is not limited to rice, dessert mixes, dried ready meals, dehydrated soup, instant soup, dried pasta, plain noodles, and instant noodles. The Chill Processed Food category includes, but is not limited to chilled processed meats, processed fish/seafood products, lunch kits, fresh cut fruits, ready meals, pizza, prepared salads, soup, fresh pasta and noodles.

The Sauces, Dressings and Condiments category includes, but is not limited to tomato pastes and purees, bouillon/stock cubes, herbs and spices, monosodium glutamate (MSG), table sauces, soy based sauces, pasta sauces, wet/cooking sauces, dry sauces/powder mixes, ketchup, mayonnaise, mustard, salad dressings, vinaigrettes, dips, pickled products, and other sauces, dressings and condiments.

The Baby Food category includes, but is not limited to milk- or soybean-based formula; and prepared, dried and other baby food.

The Spreads category includes, but is not limited to jams and preserves, honey, chocolate spreads, nut based spreads, and yeast based spreads.

The Dairy Product category generally refers to edible product produced from mammal's milk. Examples of dairy product include, but are not limited to drinking milk products, cheese, yoghurt and sour milk drinks, and other dairy products.

Additional examples for flavored products, particularly food and beverage products or formulations, are provided as follows. Exemplary ingestible compositions include one or more confectioneries, chocolate confectionery, tablets, countlines, bagged selflines/softlines, boxed assortments, standard boxed assortments, twist wrapped miniatures, seasonal chocolate, chocolate with toys, alfajores, other chocolate confectionery, mints, standard mints, power mints, boiled sweets, pastilles, gums, jellies and chews, toffees, caramels and nougat, medicated confectionery, lollipops, liquorice, other sugar confectionery, bread, packaged/industrial bread, unpackaged/artisanal bread, pastries, cakes, packaged/industrial cakes, unpackaged/artisanal cakes, cookies, chocolate coated biscuits, sandwich biscuits, filled biscuits, savory biscuits and crackers, bread substitutes, breakfast cereals, rte cereals, family breakfast cereals, flakes, muesli, other cereals, children's breakfast cereals, hot cereals, ice cream, impulse ice cream, single portion dairy ice cream, single portion water ice cream, multi-pack dairy ice cream, multi-pack water ice cream, take-home ice cream, take-home dairy ice cream, ice cream desserts, bulk ice cream, take-home water ice cream, frozen yoghurt, artisanal ice cream, dairy products, milk, fresh/pasteurized milk, full fat fresh/pasteurized milk, semi skimmed fresh/pasteurized milk, long-life/uht milk, full fat long life/uht milk, semi skimmed long life/uht milk, fat-free long life/uht milk, goat milk, condensed/evaporated milk, plain condensed/evaporated milk, flavored, functional and other condensed milk, flavored milk drinks, dairy only flavored milk drinks, flavored milk drinks with fruit juice, soy milk, sour milk drinks, fermented dairy drinks, coffee whiteners, powder milk, flavored powder milk drinks, cream, cheese, processed cheese, spreadable processed cheese, unspreadable processed cheese, unprocessed cheese, spreadable unprocessed cheese, hard cheese, packaged hard cheese, unpackaged hard cheese, yoghurt, plain/natural yoghurt, flavored yoghurt, fruited yoghurt, probiotic yoghurt, drinking yoghurt, regular drinking yoghurt, probiotic drinking yoghurt, chilled and shelf-stable desserts, dairy-based desserts, soy-based desserts, chilled snacks, fromage frais and quark, plain fromage frais and quark, flavored fromage frais and quark, savory fromage frais and quark, sweet and savory snacks, fruit snacks, chips/crisps, extruded snacks, tortilla/corn chips, popcorn, pretzels, nuts, other sweet and savory snacks, snack bars, granola bars, breakfast bars, energy bars, fruit bars, other snack bars, meal replacement products, slimming products, convalescence drinks, ready meals, canned ready meals, frozen ready meals, dried ready meals, chilled ready meals, dinner mixes, frozen pizza, chilled pizza, soup, canned soup, dehydrated soup, instant soup, chilled soup, hot soup, frozen soup, pasta, canned pasta, dried pasta, chilled/fresh pasta, noodles, plain noodles, instant noodles, cups/bowl instant noodles, pouch instant noodles, chilled noodles, snack noodles, canned food, canned meat and meat products, canned fish/seafood, canned vegetables, canned tomatoes, canned beans, canned fruit, canned ready meals, canned soup, canned pasta, other canned foods, frozen food, frozen processed red meat, frozen processed poultry, frozen processed fish/seafood, frozen processed vegetables, frozen meat substitutes, frozen potatoes, oven baked potato chips, other oven baked potato products, non-oven frozen potatoes, frozen bakery products, frozen desserts, frozen ready meals, frozen pizza, frozen soup, frozen noodles, other frozen food, dried food, dessert mixes, dried ready meals, dehydrated soup, instant soup, dried pasta, plain noodles, instant noodles, cups/bowl instant noodles, pouch instant noodles, chilled food, chilled processed meats, chilled fish/seafood products, chilled processed fish, chilled coated fish, chilled smoked fish, chilled lunch kit, chilled ready meals, chilled pizza, chilled soup, chilled/fresh pasta, chilled noodles, oils and fats, olive oil, vegetable and seed oil, cooking fats, butter, margarine, spreadable oils and fats, functional spreadable oils and fats, sauces, dressings and condiments, tomato pastes and purees, bouillon/stock cubes, stock cubes, gravy granules, liquid stocks and fonds, herbs and spices, fermented sauces, soy based sauces, pasta sauces, wet sauces, dry sauces/powder mixes, ketchup, mayonnaise, regular mayonnaise, mustard, salad dressings, regular salad dressings, low fat salad dressings, vinaigrettes, dips, pickled products, other sauces, dressings and condiments, baby food, milk formula, standard milk formula, follow-on milk formula, toddler milk formula, hypoallergenic milk formula, prepared baby food, dried baby food, other baby food, spreads, jams and preserves, honey, chocolate spreads, nut-based spreads, and yeast-based spreads. Exemplary ingestible compositions also include confectioneries, bakery products, ice creams, dairy products, sweet and savory snacks, snack bars, meal replacement products, ready meals, soups, pastas, noodles, canned foods, frozen foods, dried foods, chilled foods, oils and fats, baby foods, or spreads or a mixture thereof. Exemplary ingestible compositions also include breakfast cereals, sweet beverages or solid or liquid concentrate compositions for preparing beverages, ideally so as to enable the reduction in concentration of previously known saccharide sweeteners, or artificial sweeteners.

Some embodiments provide a chewable composition that may or may not be intended to be swallowed. In some embodiments, the chewable composition may be gum, chewing gum, sugarized gum, sugar-free gum, functional gum, bubble gum including compounds as disclosed and described herein, individually or in combination.

Typically at least a sweet receptor modulating amount, a sweet receptor ligand modulating amount, a sweet flavor modulating amount, a sweet flavoring agent amount, a sweet flavor enhancing amount, or a therapeutically effective amount of one or more of the present compounds will be added to the ingestible composition, optionally in the presence of sweeteners so that the sweet flavor modified ingestible composition has an increased sweet taste as compared to the ingestible composition prepared without the compounds of the present invention, as judged by human beings or animals in general, or in the case of formulations testing, as judged by a majority of a panel of at least eight human taste testers, via procedures commonly known in the field.

In some embodiments, compounds as disclosed and described herein, individually or in combination, modulate the sweet taste or other taste properties of other natural or synthetic sweet tastants, and ingestible compositions made therefrom. In one embodiment, the compounds as disclosed and described herein, individually or in combination, may be used or provided in its ligand enhancing concentration(s). For example, the compounds as disclosed and described herein, individually or in combination, may be present in an amount of from 0.001 ppm to 100 ppm, or narrower alternative ranges from 0.1 ppm to 50 ppm, from 0.01 ppm to 40 ppm, from 0.05 ppm to 30 ppm, from 0.01 ppm to 25 ppm, or from 0.1 ppm to 30 ppm, or from 0.1 ppm to 25 ppm, or from 1 ppm to 30 ppm, or from 1 ppm to 25 ppm.

In some embodiments, the ingestible compositions disclosed herein, individually or in combination, may be provided in a flavoring concentrate formulation, e.g., suitable for subsequent processing to produce a ready-to-use (i.e., ready-to-serve) product. By "a flavoring concentrate formulation", it is meant a formulation which should be reconstituted with one or more diluting medium to become a ready-to-use composition. The term "ready-to-use composition" is used herein interchangeably with "ingestible composition", which denotes any substance that, either alone or together with another substance, can be taken by mouth whether intended for consumption or not. In one embodiment, the ready-to-use composition includes a composition that can be directly consumed by a human or animal. The flavoring concentrate formulation is typically used by mixing with or diluted by one or more diluting medium, e.g., any consumable or ingestible ingredient or product, to impart or modify one or more flavors to the diluting medium. Such a use process is often referred to as reconstitution. The reconstitution can be conducted in a household setting or an industrial setting. For example, a frozen fruit juice concentrate can be reconstituted with water or other aqueous medium by a consumer in a kitchen to obtain the ready-to-use fruit juice beverage. In another example, a soft drink syrup concentrate can be reconstituted with water or other aqueous medium by a manufacturer in large industrial scales to produce the ready-to-use soft drinks. Since the flavoring concentrate formulation has the flavoring agent or flavor modifying agent in a concentration higher than the ready-to-use composition, the flavoring concentrate formulation is typically not suitable for being consumed directly without reconstitution. There are many benefits of using and producing a flavoring concentrate formulation. For example, one benefit is the reduction in weight and volume for transportation as the flavoring concentrate formulation can be reconstituted at the time of usage by the addition of suitable solvent, solid or liquid.

The flavored products set forth according to any of the foregoing embodiments, also include, in certain embodiments, one or more additional flavor-modifying compounds, such as compounds that enhance sweetness (e.g., hesperetin, naringenin, glucosylated steviol glycosides, etc.), compounds that block bitterness, compounds that enhance umami, compounds that reduce sourness, compounds that enhance saltiness, compounds that enhance a cooling effect, or any combinations of the foregoing.

**In** certain embodiments of any aspects and embodiments set forth herein that refer to a sweetening or flavoring concentrate, the sweetening or flavoring concentrate is a non-naturally-occurring product, such as a composition specifically manufactured for the production of a flavored product, such as food or beverage product.

In one embodiment, the flavoring concentrate formulation comprises i) compounds as disclosed and described herein, individually or in combination; ii) a carrier; and iii) optionally at least one adjuvant. The term "carrier" denotes a usually inactive accessory substance, such as solvents, binders, or other inert medium, which is used in combination with the present compound and one or more optional adjuvants to form the formulation. For example, water or starch can be a carrier for a flavoring concentrate formulation. In some embodiments, the carrier is the same as the diluting medium for reconstituting the flavoring concentrate formulation; and in other embodiments, the carrier is different from the diluting medium. The term "carrier" as used herein includes, but is not limited to, ingestibly acceptable carrier.

The term "adjuvant" denotes an additive which supplements, stabilizes, maintains, or enhances the intended function or effectiveness of the active ingredient, such as the compound of the present invention. In one embodiment, the at least one adjuvant comprises one or more flavoring agents. The flavoring agent may be of any flavor known to one skilled in the art or consumers, such as the flavor of chocolate, coffee, tea, mocha, French vanilla, peanut butter, chai, or combinations thereof. In another embodiment, the at least one adjuvant comprises one or more sweeteners. The one or more sweeteners can be any of the sweeteners described in this application. In another embodiment, the at least one adjuvant comprises one or more ingredients selected from the group consisting of a emulsifier, a stabilizer, an antimicrobial preservative, an antioxidant, vitamins, minerals, fats, starches, protein concentrates and isolates, salts, and combinations thereof. Examples of emulsifiers, stabilizers, antimicrobial preservatives, antioxidants, vitamins, minerals, fats, starches, protein concentrates and isolates, and salts are described in U.S. Pat. No. 6,468,576, the content of which is hereby incorporated by reference in its entirety for all purposes.

In one embodiment, the present flavoring concentrate formulation can be in a form selected from the group consisting of liquid including solution and suspension, solid, foamy material, paste, gel, cream, and a combination thereof, such as a liquid containing certain amount of solid contents. In one embodiment, the flavoring concentrate formulation is in form of a liquid including aqueous-based and nonaqueous-based. In some embodiments, the present flavoring concentrate formulation can be carbonated or non-carbonated.

The flavoring concentrate formulation may further comprise a freezing point depressant, nucleating agent, or both as the at least one adjuvant. The freezing point depressant is an ingestibly acceptable compound or agent which can depress the freezing point of a liquid or solvent to which the compound or agent is added. That is, a liquid or solution containing the freezing point depressant has a lower freezing point than the liquid or solvent without the freezing point depressant. In addition to depress the onset freezing point, the freezing point depressant may also lower the water activity of the flavoring concentrate formulation. The examples of the freezing point depressant include, but are not limited to, carbohydrates, oils, ethyl alcohol, polyol, e.g., glycerol, and combinations thereof. The nucleating agent denotes an ingestibly acceptable compound or agent which is able to facilitate nucleation. The presence of nucleating agent in the flavoring concentrate formulation can improve the mouthfeel of the frozen Blushes of a frozen slush and to help maintain the physical properties and performance of the slush at freezing temperatures by increasing the number of desirable ice crystallization centers. Examples of nucleating agents include, but are not limited to, calcium silicate, calcium carbonate, titanium dioxide, and combinations thereof.

In one embodiment, the flavoring concentrate formulation is formulated to have a low water activity for extended shelf life. Water activity is the ratio of the vapor pressure of water in a formulation to the vapor pressure of pure water at the same temperature. In one embodiment, the flavoring concentrate formulation has a water activity of less than about 0.85. In another embodiment, the flavoring concentrate formulation has a water activity of less than about 0.80. In another embodiment, the flavoring concentrate formulation has a water activity of less than about 0.75.

In one embodiment, the flavoring concentrate formulation has the present compound in a concentration that is at least 2 times of the concentration of the compound in a ready-to-use composition. In one embodiment, the flavoring concentrate formulation has the present compound in a concentration that is at least 5 times of the concentration of the compound in a ready-to-use composition. In one embodiment, the flavoring concentrate formulation has the present compound in a concentration that is at least 10 times of the concentration of the compound in a ready-to-use composition. In one embodiment, the flavoring concentrate formulation has the present compound in a concentration that is at least 15 times of the concentration of the compound in a ready-to-use composition. In one embodiment, the flavoring concentrate formulation has the present compound in a concentration that is at least 20 times of the concentration of the compound in a ready-to-use composition. In one embodiment, the flavoring concentrate formulation has the present compound in a concentration that is at least 30 times of the concentration of the compound in a ready-to-use composition. In one embodiment, the flavoring concentrate formulation has the present compound in a concentration that is at least 40 times of the concentration of the compound in a ready-to-use composition. In one embodiment, the flavoring concentrate formulation has the present compound in a concentration that is at least 50 times of the concentration of the compound in a ready-to-use composition. In one embodiment, the flavoring concentrate formulation has the present compound in a concentration that is at least 60 times of the concentration of the compound in a ready-to-use composition. In one embodiment, the flavoring concentrate formulation has the present compound in a concentration that is up to 100 times of the concentration of the compound in a ready-to-use composition.

In some embodiments, the flavorings may be used in many distinct physical forms well- known in the art to provide an initial burst of flavor and/or a prolonged sensation of flavor. Without being limited thereto, such physical forms include free forms, such as spray dried, powdered, beaded forms, encapsulated forms, and mixtures thereof.

Suitable bulking agents include, but are not limited to maltodextrin (10 DE, 18 DE, or 5 DE), corn syrup solids (20 or 36 DE), sucrose, fructose, glucose, invert sugar, sorbitol, xylose, ribulose, mannose, xylitol, mannitol, galactitol, erythritol, maltitol, lactitol, isomalt, maltose, tagatose, lactose, inulin, glycerol, propylene glycol, polyols, polydextrose, fructooligosaccharides, cellulose and cellulose derivatives, and the like, and mixtures thereof. Additionally, granulated sugar (sucrose) or other caloric sweeteners such as crystalline fructose, other carbohydrates, or sugar alcohols can be used as a bulking agent due to their provision of good content uniformity without the addition of significant calories.

In one embodiment, the at least one bulking agent may be a bulking agent described in U.S. Patent No. 8,993,027.

In one embodiment, the at least one bulking agent may be a bulking agent described in U.S. Patent No. 6,607,771.

In one embodiment, the at least one bulking agent may be a bulking agent described in U.S. Patent No. 6,932,982.

In some embodiments, the tabletop sweetener composition may further comprise at least one anti-caking agent. As used herein the phrase "anti-caking agent" and "flow agent" refer to any composition which prevents, reduces, inhibits, or suppresses the at least one sweetener from attaching, binding, or contacting to another sweetener molecule. Alternatively, anti-caking agent may refer to any composition which assists in content uniformity and uniform dissolution. Non-limiting examples of anti-caking agents include cream of tartar, calcium silicate, silicon dioxide, microcrystalline cellulose (Avicel, FMC BioPolymer, Philadelphia, Pa.), and tricalcium phosphate. In one embodiment, the anti-caking agents are present in the tabletop sweetener composition in an amount from about 0.001 to about 3% by weight of the tabletop sweetener composition.

In some embodiments, the sweetener compositions of any of the preceding aspects and embodiments thereof are encapsulated using typical means for encapsulating flavor or fragrance compounds. Non-limiting examples of such technology are set forth in U.S. Patent Application Publication Nos. 2016/0235102, 2019/0082727, 2018/0369777, 2018/0103667, 2016/0346752, 2015/0164117, 2014/0056836, 2012/0027866, 2010/0172945, and 2007/0128234, as well as U.S. Patent Nos. 7,488,503, 6,416,799, 5,897,897, 5,786,017, 5,603,971, 4,689,235, 4,610,890, 3,704,137, 3,041,180, and 2,809,895.

### EXAMPLES

To further illustrate this invention, the following examples are included. The examples should not, of course, be construed as specifically limiting the invention. Variations of these examples within the scope of the claims are within the purview of one skilled in the art and are considered to fall within the scope of the invention as described, and claimed herein. The reader will recognize that the skilled artisan, armed with the present disclosure, and skill in the art is able to prepare and use the invention without exhaustive examples.

### Example 1 - Synthesis of MC1 Compound

The UDP-glycotransferase 85 (UGT85) is used to make the MC1 compound from the alpha-1,6 isomer of siamenoside I (siratose). The UGT85 sequence is set forth in Itkin et al., PROC. NAT'L ACAD. SCI. USA, vol. 113(47), pp. E7619-E7628 (2016). In other words, a single beta-D-glucose unit is added to the 4-position of the lone glucose moiety attached to the 3-position of the cucurbitane backbone. The additional glucose unit is added via a beta connection at its 1-position. The gene from *Siraitia grosvenorii* was overexpressed in insect cell line SF9 using pFastBac1 vector. For the reaction, 1 g (500 mg/L final concentration) of siratose was reacted with 70 mL of UGT85 crude extract, 10 mL crude extract containing sucrose synthase, 1.7 g UDP-Glucose, 1 ml 100x protease inhibitor, 103 g sucrose, 0.2 g G418 antibiotic, 5 mM MgCl, 5 mM KCl, in 0.1 M Tris-HCl pH 8.0, incubated at 30 °C for 24 hrs while stirring (450 rpm). The reactions were stopped by heating to 80°C and adding equal volume ethanol. The supernatant was collected by centrifugation (15000 x g, 10min) and vacuum filtered (0.2um). The sample was analyzed by UPLC (C18 column, 10 to 50, 6 minutes). The protein and DNA sequence are provided below.

The enzyme reaction was scaled up to 1.5 g by repeating 2 more times. Each of the 3 filtrates from 3 separate reactions was concentrated to ~100 mL size and loaded onto a 60 g C18 SPE cartridge with a 10 g samplet cartridge and fractionate on SPE using 5, 15, 95 and 100% methanol/water step gradient. The desired compound was eluted in 95, 100% methanol steps. The combined fractions from three runs were dried on rocket and Rotovap followed by HPLC purification on 3x10cm C18 column using 2-20% acetonitrile/water method for 31 minutes at 40mL/min flow rate. The desired compound was eluted in fractions 13 and 14. The combined fractions 13 and 14 were dried on Genevac to get 438 mg with about 80% purity. This fraction was further purified on 3x10cm HILIC Amide column using 75 to 55% B (mobile phase, A=3:1 methanol:water, B = acetonitrile) for about 42 minutes at 30 mL/min flow rate. The fractions eluted with desired compound with desired purity were pooled and dried down on a clean Genevac, re-suspended in 5 mL of water and lyophilized for about a week to get 80 mg of >95% pure compound.

The identity of the product was confirmed by NMR and mass spec data. ¹H NMR (400 MHz, Pyridine-ds) data of key signals δ 5.40 (m, 3H, H-6, G2 and G3-1H, merged with HDO peak), 5.15 (d, *J* = 7.9 Hz, 1H, G5-1H), 4.87 (d, *J =* 7.7 Hz, 1H, G1-1H), 4.77 (d, *J* = 7.8 Hz, 1H, G4-1H), 3.72 (d, *J =* 8.2 Hz, 1H, 24-H), 3.57 (s, 1H, 3-H), 1.48 (s, 3H, 28-Me), 1.42 (s, 3H, 26-Me), 1.30 (s, 3H, 27-Me), 1.27 (s, 3H, 19-Me), 1.10 (s, 3H, 29-Me), 1.02 (d, *J* = 6.2 Hz, 3H, 21-Me), 0.87 (s, 3H, 30-Me), 0.84 (s, 3H, 18-Me). The mass was also confirmed via mass spectrometry: ESI-MS 1287.9 (MH)⁺, 1285.5 (M-H)⁻.

### Example 2 - Assay Testing with Sweet Receptor Assays

Cells stably expressing the recombinant human sweet receptor and the chimeric G-protein G16g25 were dispensed in a 384-well clear bottom plate (Fisher) and incubated overnight at 37 °C and 6% CO₂. On the day of the experiment, cells were loaded with 4 µM of the calcium indicator Fluo-4 AM (Invitrogen) in D-PBS for 1 hour at room temperature and excess dye washed away with D-PBS, leaving a residual volume of 25 µL/well. After 30 minutes of rest time the cell plate and the compound plate were transferred into a fluorometric imaging plate reader (Molecular Devices). To determine potency, compound plates were prepared with serial dilutions of compound at 2 x the final assay concentration in D-PBS. Imaging and data analysis was performed according to the procedures set forth in Servant et al., PROC. NAT'L ACAD. SCI. USA, vol. 107(10), pp. 4746-51 (2010). When tested in the sweet taste assays, the MC1 compound showed a dose-response effect indicative of its utility as a sweetener, where the dose-response curve showed an EC₅₀ of 8.0 µM.

## Claims

1. A sweetening compound, wherein the sweetening compound is a compound of formula (I) 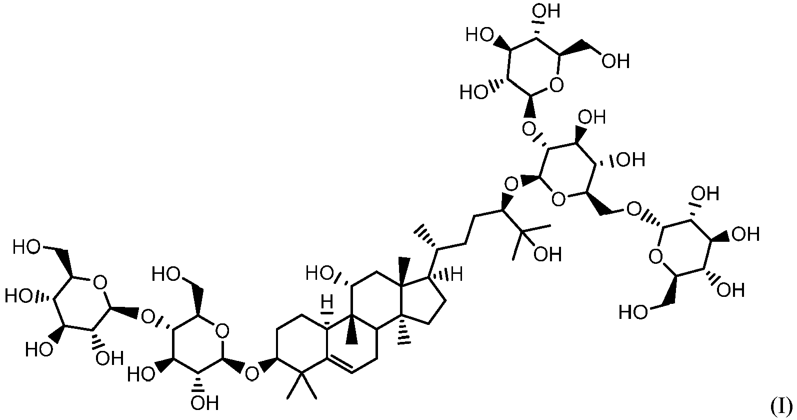 or a comestibly acceptable salt thereof.

2. The sweetening compound of claim 1, wherein the sweetening compound is the compound of formula (I).

3. Use of a compound to sweeten an ingestible composition, wherein the compound is a sweetening compound of claim 1 or 2.

4. The use of claim 3, wherein the concentration of the sweetening compound used in the ingestible composition ranges from 0.1 ppm to 1000 ppm.

5. The use of claim 3 or 4, wherein the ingestible composition further comprises monk fruit juice, monk fruit extract, or any combination thereof.

6. The use of any one of claims 3 to 5, wherein the ingestible composition comprises one or more bitter tastants.

7. An ingestible composition, which comprises a sweetening compound of claim 1 or 2.

8. The ingestible composition of claim 7, wherein the sweetening compound is present in the ingestible composition at a concentration ranging from 0.1 ppm to 1000 ppm.

9. A flavored product, which comprises the ingestible composition of claim 7 or 8.

10. The flavored product of claim 9, which is a beverage product, a food product, or an oral care product.

## Patentansprüche

1. Süßende Verbindung, wobei die süßende Verbindung eine Verbindung der Formel (I) 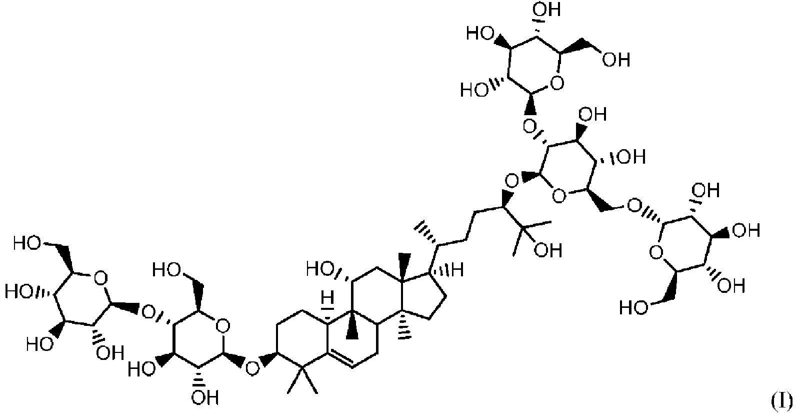 oder ein lebensmittelgeeignetes Salz davon ist.

2. Süßende Verbindung nach Anspruch 1, wobei die süßende Verbindung die Verbindung der Formel (I) ist.

3. Verwendung einer Verbindung zum Süßen einer verzehrbaren Zusammensetzung, wobei die Verbindung eine süßende Verbindung nach Anspruch 1 oder 2 ist.

4. Verwendung nach Anspruch 3, wobei die Konzentration der in der verzehrbaren Zusammensetzung verwendeten süßende Verbindung in dem Bereich von 0,1 ppm bis 1000 ppm liegt.

5. Verwendung nach Anspruch 3 oder 4, wobei die verzehrbare Zusammensetzung ferner Mönchsfruchtsaft, Mönchsfruchtextrakt oder eine beliebige Kombination davon umfasst.

6. Verwendung nach einem der Ansprüche 3 bis 5, wobei die verzehrbare Zusammensetzung einen oder mehrere bitter schmeckende Stoffe umfasst.

7. Verzehrbare Zusammensetzung, die eine süßende Verbindung nach Anspruch 1 oder 2 umfasst.

8. Verzehrbare Zusammensetzung nach Anspruch 7, wobei die süßende Verbindung in der verzehrbaren Zusammensetzung in einer Konzentration in dem Bereich von 0,1 ppm bis 1000 ppm vorhanden ist.

9. Aromatisiertes Produkt, das die verzehrbare Zusammensetzung nach Anspruch 7 oder 8 umfasst.

10. Aromatisiertes Produkt nach Anspruch 9, das ein Getränkeprodukt, ein Lebensmittelprodukt oder ein Mundpflegeprodukt ist.

## Revendications

1. Composé édulcorant, dans lequel le composé édulcorant est un composé de formule (I) 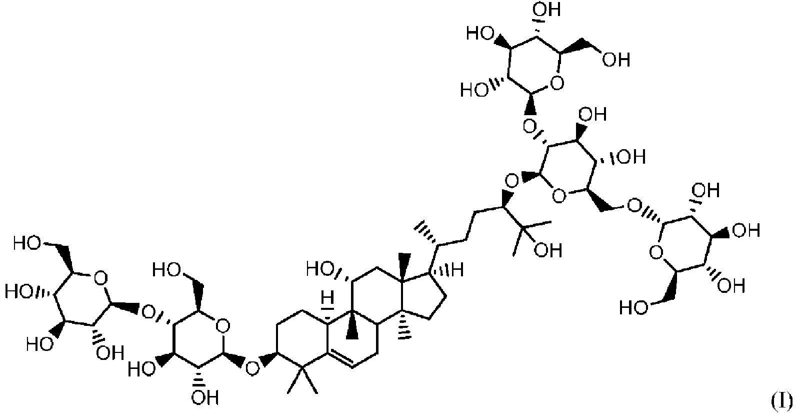 ou un sel acceptable de celui-ci sur le plan du caractère comestible.

2. Composé édulcorant selon la revendication 1, dans lequel le composé édulcorant est le composé de formule (I).

3. Utilisation d'un composé pour édulcorer une composition ingérable, dans laquelle le composé est un composé édulcorant selon la revendication 1 ou 2.

4. Utilisation selon la revendication 3, dans laquelle la concentration du composé édulcorant utilisé dans la composition ingérable est dans la plage de 0,1 ppm à 1 000 ppm.

5. Utilisation selon la revendication 3 ou 4, dans laquelle la composition ingérable comprend en outre du jus de fruit de moine, de l'extrait de fruit de moine ou toute combinaison de ceux-ci.

6. Utilisation selon l'une quelconque des revendications 3 à 5, dans laquelle la composition ingérable comprend un ou plusieurs agents de goût amers.

7. Composition ingérable, qui comprend un composé édulcorant selon la revendication 1 ou 2.

8. Composition ingérable selon la revendication 7, dans laquelle le composé édulcorant est présent dans la composition ingérable à une concentration dans la plage de 0,1 ppm à 1 000 ppm.

9. Produit aromatisé, qui comprend la composition ingérable selon la revendication 7 ou 8.

10. Produit aromatisé selon la revendication 9, qui est une boisson, un produit alimentaire ou un produit de soins buccaux.
